# EUROPEAN PATENT APPLICATION

(11) **EP 0 875 594 A1**
(43) Date of publication of application: **04.11.1998**
(21) Application number: 97911242.2
(22) Date of filing: 10.11.1997
(51) Int. Cl.: C23C 14/28, C23C 14/06, C23C 14/02, A61F 2/30, A61L 27/00

(54) **METHOD FOR IMPROVING THE OSTEOINTEGRATION OF OSSEOUS FIXING IMPLANTS**

(30) Priority: 12.11.1996 ES 9602439
(71) Applicant: UNIVERSIDAD DE VIGO, 36280 Vigo (ES); Erothitan Titanimplantate AG i.G., 98574 Schmalkalden (DE); Euro TRS, 01005 Vitoria (ES); Klockner, 08030 Barcelona (ES); Implantes AB, 08201 Sabadell (ES); Bone System, 20134 Milano (IT)
(72) Inventor: LEON FONG, Betty Mireya, Lagoas-Marcosende, 9 E-36280 Vigo (ES); POU SARACHO, Juan Maria, Lagoas-Marcosende, 9 E-36280 Vigo (ES); ARIAS OTERO, Jorge Luis, Lagoas-Marcosende, 9 E-36280 Vigo (ES); GARCIA SANZ, Francisco Jose, Lagoas-Marcosende, 9 E-36280 Vigo (ES); MAYOR LEIROS, Mercedes Belén, Lagoas-Marcosende, 9 E-36280 Vigo (ES); GONZALEZ FERNANDEZ, Pio Manuel, Lagoas-Marcosende, 9 E-36280 Vigo (ES); PEREZ-MARTINEZ Y PEREZ-AMOR, Mariano Jesús, Lagoas-Marcosende, 9 E-36280 Vigo (ES)
(74) Representative: Urizar Anasagasti, José Antonio
(86) International application number: PCT/ES97/00269
(87) International publication number: WO 98/21380

(57) **Abstract**

The osteointegration of osseous fixing implants may be improved through a series of surface treatments. Said treatments comprise a cleaning and passivation process, tha application of a coating in the system Ca-P-H-O-C through the laser ablation technique and a sterilization process by irradiation. The flexibility of this method provides for the control of physico-chemical properties of the obtained surface in order to match said properties with those of the bone in which the implant will be placed.

## Description

The present invention refers to a method for improving the osteointegration of implants for orthopaedia and odontology applications.

This invention provides a series of treatments on osseus fixing implant surface, such that the implant subject to said treatment improves substantially its osteointegration properties with respect to the bone with which the implant will be in contact. These treatments comprise a cleaning and passivation process, the application of a Ca-P-H-O-C system coating (i.e., a Ca_{U}P_{V}H_{X}O_{Y}C_{Z}, where subindexes u, v, x, y, z are natural numbers including zero) by means of the laser ablation technique and an sterilisation by irradiation process.

When the replacement of a defective member of a human body is required, the best solution would be the use of compatible tissue or organ provided by a human donor, since natural tissues and organs contain the light proportions of materials needed to satisfy the body functions. However, beacause of increasing demand for transplants, there are not enough human donors to satisfy the need of living implants. On the other hand, when natural implants are used, frequently rejection problems appear in the receiving body.

A solution to meet said increasing demand is the use of manmade tissues and organs. Since 1950 a continuous research has been made on biomaterials and non-farmacological products that are adequate to improve or substitute the functions of human organs and tissues.

Present biomaterials are useful for many applications, from substitution of intraocular lenses to the manufacture of artificial hearts. Generally, a biomaterial can be defined as a mechanically and biologically compatible material, i.e., it must stand the mechanical stresses derived from its function, and be non toxic, controllable y predictable on its interaction with human body.

In particular, a set of specifications can be defined, which the biomaterials should meet (see, for example: S.F. Hulbert "Use of Ceramics in Surgical Implants", ed. By S.F. Hulbert and F.A. Young. Gordon and Breach Science Publishers, New York (1969) 1):
a) be resistant to the action of body fluids; b) stand the mechanical stresses proper of the required functions; c) be adaptable to the required shape; d) should not generate toxic or allergenic reactions; e) should not affect the body defense natural mechanisms; f) should not help to the formation of blood platelets, or to the coagulation or denaturalization of plasma proteins.
In other words, the material needs to be compatible from both points of view: the effects of human body on the implant and the effect of said implant (or substances produced due to corrossion or wear) onto human body.

The most used odontological and orthopaedic implants are those made of pure titanium or with titanium alloys (Ti-6Al-4V) (D.F. Williams, J. Med. Eng. Technol. I (1977) 266-270) for they enjoy a high resistance to mechanical stresses together with a resistance to corrossion; this property is due to its high affinity to oxygen, generating a five protective oxide coating at ambient temperature. However this affinity with oxygen makes particularly difficult the cleaning of said material.

Since titanium and tintanium alloys implants are bioinert implants, in case that an interface displacement occurred during the recovery period, the fibrous tissue capsule may be too thick, this causing a quick loosening of the implant and, finally, breaking the implant or the adjacent bone (L.L. Hench & J. Wilson, "An introduction to Bioceramics", ed. By L.L. Hench & J. Wilson, Advance Series in Ceramics-Vol.1. World Scientific Publishing, Singapore, (1933) 1-24). Furthermore, the metal implants widely differ, in their mechanical features as well as in their composition, from osseous tissues where they are to be inserted.

With the purpose to avoid the disadvantages of metal implants, tests have been conducted on non metal materials such as ceramics, polymers and hybrid materials.

Since the bone mineral phase is made of calcium phosphate, researches have been conducted on biocompatible ceramics, and in particular on different types of calcium phosphates (CaP) such as hydroxyapatite, tricalcium phosphate, carbonated hydroxyapatite, apatite, pyrophosphate, tetracalcium phosphate, etc. The advantage of this type of material is based on the direct coupling to the bone with no fibrous tissue in the interface, i.e., they have a high bioactivity (S. Best "seminar in Bio-Active Materials in Orthopaedics" Sep. 27, 1994, Cambridge, UK).

Among these calcium phosphates, the hydroxyapatite (HA), having the formula Ca₁₀(PO₄)₆(OH)₂, has generated a great interest since for a long time it was believed that it had the same composition as the calcium phosphate of bone. At present it is widely accepted that bone develops a strong coupling with implants made with sintered hydroxyapatite (HA) (K. DeGroot, R. Geesink, C.P.A.T.Klein and P. Serekian, Journal of Biomedical Materials Research, Vol. 21, (1987) 1375-1381), i.e., hydroxyapatite has a very high bioactivity.

The main disadvantage in the use of implants made of said calcium phosphates is the low resistance to mechanical stresses due to its poor mechanical properties. For this reason the application of calcium phosphate coatings over metal substrates or other materials subject to stresses is becoming very common for odontological and orthopaedic implants.

The CaP coatings more frequently applied on odontological and orthopaedic implants are those made of HA. If the HA coating is pure and thick, i.e., with low porosity, it will not be reabsorbed and the coupling between bone and HA will take place in a 3-6 week period. The HA density will depend on the morphology and phisical and chemical properties of the HA powder used. The coupling between bone and hydroxyapatite has been proved by tests conducted on animals as well as on human (R.G.T. Geesink, Ph. D. Thesis, Marquette University, 1974). Furthermore, hydroxypatite is considered to be an osteoconductive material, meaning that it would force the bone growth in accordance with HA own crystal structure.

However, it has been found recently that the CaP composition, forming the bone mineral phase, is not exactly the same as for the HA, for it contains carbon in carbonate groups to constitute a carbonated hydroxyapatite (HAC). Also, it has been found that the interface between implants made of different CaP and the bone is made of said hydroxyapatite (HAC). The crystal structure of this HAC coincides in practice with that of HA. For this reason, it is thought that this HAC can be a much more bioactive ceramic than HA and, possibly, with a higher osteoconductivity. In fact, HAC powders are being produced and sintered for use in implants.

The methods commonly used to produce such coatings are the electrophoresis deposition (P. Ducheyne, W.V. Raemdonck, J.C. Heughebaert & M. Heughebaetrt, Biomaterials 7 (1986) 97), the plasma spraying (S.D. Cook, J.F. Kay, K.A. Thomas, R.C. Anderson, M.C. Reynolds & J. Jarcho, J. Dental Res. 65 (1986) 222) and the cathodic spraying by radio frequency (E. Ruckenstein, S. Gourisanker & R.E. Baier, J. Colloid abd Interface Sci. 63 (1983) 245). The method most commonly used to produce such HA coatings is the plasma spraying method, which creates 50-200µm thick coatings with a HA content of 90%, in case that the initiation material is 100% HA. The minimum thickness obtained by industrial process is 50-60 µm and it is the result of a compromise between minimum thickness and homogeneity, due to the fact that this technique produces very high porosity coatings and, thus, it becomes necessary to deposit sucessive coatings of approximate 10 µm thickness in order to obtain an uniform granular coating. However, such coatings present following inconveniences: a) at present it is not possible to get continuous coatings of less than 20 µm thickness; b) the coatings have a granular morphology, i.e. they have a high porosity; c) due to the coating thickness (50-200 µm) the adherence to the substrate is very poor; d) as a result of the stresses produced, due to the difference of elongation degree in substrate and coating, frequent cracks appear; e) some coating areas turn out to be amorphic or microcrystalline (microcrystals of tricalcium phosphate) due to plasma high temperatures, thus generating the breakdown and excessive fusion of HA powders. For this reason, they are highly absorbable, with difficulty in controlling the absorbability degree.

In practice, and due to flaking problems (low adehrence substrate-coating), many surgeons prefer to use, in odontological and orthopaedic applications, non coated implants to avoid the high risk of coating failure.

The laser ablation technique used in the present invention was fist used in 1965, only five years after the construction of the first ruby laser. Since then, this technique has been used for the production of coatings with all types of materials (see for example: D.-B. Chrisey & G.K Hubler ed. "Pulsed laser deposition of thin films". John Wiley and Sons, Inc. New York (1994)). The present invention includes an application of said technique to the field of osteointegrated coatings.

One of the advantages of present invention is the possibility to apply a gradual composition coating in the Ca-P-H-O-C system, with the option to adapt said coating to the features of the implant or to those of bone in contact with the implant. The coating may have a composition such that reabsorption rate by the bone is equal to the bone growth rate, thus obtaining an optimum coupling in every moment, and therefore finally providing a perfect osteointegrated implant.

Another advantage of the method object of the present invention consists of a substantial improvement in the adherence between coating and implant, thus avoiding one of the greatest disadvantages of coatings applied by means of the plasma spraying technique.

Furthermore, the method object of present invention does not affect to implant properties since the application temperature is not too high (450 °C) and relatively low compared to very high temperatures reached with the plasma used in the plasma spraying technique.

It must, also, be noted that the coatings applied have a thickness of several microns, then not being necessary to apply any further treatment after deposition (except for sterilization). Therefore, the element treated does not require any further machining, thus reducing the residual stresses that can generate cracks.

The method for improving the osteointegration of osseous fixing implants, object of present invention, consists of sucessive treatments of implant surface, so that the final result is the improvement of the osteointegration when implanted in the bone.

To better understand the object of present invention a preferred practical embodiment of the method for improving the osteointegration of osseous fixing implants, is described hereinafter, and in accordance with enclosed figure where a vacuum chamber is shown to be used for that purpose.

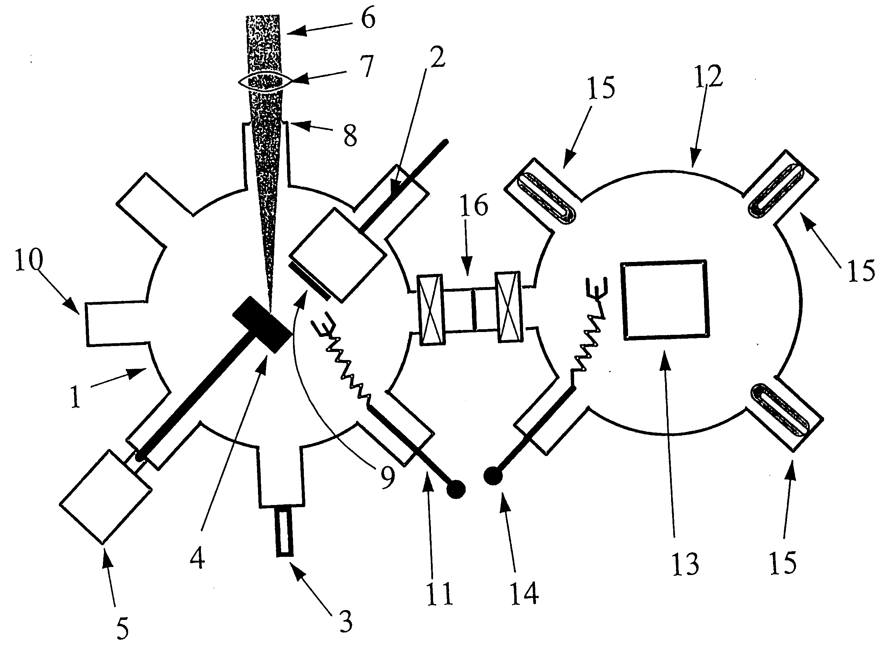

The process is essentially conducted in a vacuum chamber, as shown in the figure. First, the implant, once manufactured, has to be subject to a cleaning and passivation treatment. This process can be conducted with acid attack in an ultrasounds bath.

Once the cleaning and passivation process is finished, the implant is placed inside a vacuum chamber (1) specifically designed for this process. The basic material used for the coating (4) is placed inside the vacuum chamber (1) and can be rotated during the deposition process by means of a motor (5). Said vacuum chamber (1) should previously be connected to the vacuum control system, to the controlled flow gas feeding system and to the temperature control system, which are commonly used in industrial equipments and, therefore, not shown in the figure. Once this vacuum chamber (1) is closed, a vacuum is applied, typically 10⁻⁸ torr.(133.3x10⁻⁶ Pa) and the implant is moderately heated by means of an appropiate heater (2) integrated inside the vacuum chamber (1). When the required pressure and temperature are reached, said chamber will be filled with an atmosphere where the deposition process will take place. Said atmosphere will depend on the type of material required, and may consist of Ar, H₂, O, O₂, CO₂, or mixtures thereof. The gases are supplied by means of the gas feeding system (3). Once the required gas composition as well as the required temperature are obtained, the basic material used for the coating (4) is subject to ultraviolet radiation provided by an excimere laser (6). The ultraviolet radiation enters the chamber (1) through a transparent window (8) (for example molten silica). With the purpose to obtain a high density of ultraviolet radiation energy inside the chamber (1), the laser beam (6) can be focused by means of a lens (7). Due to the laser beam radiation (6), the material used for the coating (4) is ablated and moved towards the implant (9) to create an adhesive coating covering the implant whole surface. The gases not used in the process and residual products are expelled through the gas outlet (10) and conveyed to an adequate gas treatment system (not shown in the figure).

Once the required coating is provided, the implant is conveyed by means of a vacuum handling system (11, 14 and 16) to within a second vacuum chamber (12) where the coated implant is subject to ultraviolet radiation produced by silent discharge excimer lamps (15) with the purpose to sterilize said implant as well as the container (13). Once the sterilization process is completed, the implant is introduced into the containmer (13) which is sealed by means of the vacuum handling system (14) and said implant is perfectly vacuum sealed and ready for application.

As an example, with the method described hereinbefore and using an excimer laser (λ = 193 nm) operating on 20 Hz pulse mode and with energy density of 2.5 J/cm², a water vapor atmosphere, total pressure = 0.45 mbar (45 Pa), temperature = 485 °C, distance coating material-implant = 0.045 m, rotation speed of coating material = 1 rpm, a coating of carbonated calcium phosphate on pure titanium implant was at least of 0.54 A/pulse with an adherence degree to the implant of at least 58 Mpa.

The method described hereinbefore can provide a very tacky Ca_{U}P_{V}H_{X}O_{Y}C_{Z} film on implants formed with a plurality of materials as, for example: titanium and its alloys, steels, Cr and Co alloys or hybrid materials.

The great flexibility provided by this method allows for the modification of the physical and chemical properties and the film thickness by only changing the process parameters. Thus, it will be possible to obtain Ca_{U}P_{V}H_{X}O_{Y}C_{Z} coatings with different optical, electrical and mechanical properties, what will be very useful to adapt the coating to specific features of the substrate and to the type of bone that will be in contact with said implant.

This method will allow for an improved osteointegration of finished implants, with a constant size, so that the parts treated by the method described in the present invention, will still meet the allowances previously specified for the implant application.

Once the nature, as well as one embodiment of the present invention have been thoroughly described, it is only to be added that it will be possible to introduce changes in the shape, materials and arrangement, as long as those changes do not substantially affect to the characteristicas of the invention as claimed hereinafter.

## Claims

1. A method for improving the osteointegration of osseous fixing implants characterized in that it comprises:
a) Cleaning and passivation of the implant by means of acid atack in an ultrasounds bath;
b) introduction of said implant in a vacuum chamber, said chamber being provided with a high vacuum control system, a heating system with temperature control, a gas flow control and feeding system, a pressure control system inside said vacuum reaction chamber and a coating material fastening and rotation system;
c) introduction of an atmosphere used in the deposition process, said atmosphere consisting of argon, water vapor, oxygen or mixtures thereof;
d) irradiation of coating material with ultraviolet photons (λ = 193 nm) provided by an ArF excimer laser, introducing said laser beam through a window transparent to said beam;
e) application of a coating in the Ca-P-H-O-C system, i.e., a coating of Ca_{U}P_{V}H_{X}O_{Y}C_{Z} where subindexes u,v,x,y,z are natural numbers including zero with variable stoichiometry on implant surface;
f) exposing the container with the implant to an ultraviolet radiations in high vacuum to sterilize the container as well as the implant;
g) introduction of implant in a sterilized container;
h) sealing of container with the implant inside to obtain a sterilized vacuum assembly;
i) pulling the container out of the vacuum chamber.

2. A method according to claim 1, wherein all or some of the procedures described in items f), g), h) and i) above can be substituted by similar procedures having the purpose of sterilizing and packing said implant in a inert atmosphere.

3. A method according to claim 1, wherein the vacuum chamber is provided with a rotating means for holding the implants to allow for an uniform coating of several implants at a time.

4. A method according to claims 1 to 3, wherein an electric field is established between the coating material and the implant.

5. A method according to claims 1 to 4, wherein the cleaning process comprises:
a) cleaning with ketone in an ultrasounds bath for 8 minutes;
b) cleaning with deionized water;
c) cleaning with HNO₃, with a 5% content, in an ultrasounds bath for 8 minutes;
d) cleaning with deionized water;
e) cleaning with methanol;
all above processes being conducted at normal pressure and temperature conditions.

6. A method according to claims 1 to 5, wherein the implant to be coated is subject to a ultraviolet radiation before and/or after introducing an atmosphere, said radiation being produced either by a laser beam, or a mercury lamp, or a silent discharge excimer lamp or any other ultraviolet radiation source such that the whole implant surface gets sterilized and chemically activated.

7. A method according to claims 1 to 6, whereinm the deposed material in the Ca-P-H-O-C system has a variable stoichiometry.

8. A method according to claims 1 to 6, wherein the deposed material consists of a double coat of material in the system Ca-P-H-O-C, where both coatings present different crystalline degrees.

9. A method according to claims 1 to 6, wherein the material is deposed with controlled preferential crystalographic orientations, producing, once the implant is applied, an epitaxial growth of the newly formed bone.

10. A method according to claims 1 to 6, wherein the material presents a crystalline or composition degree such that the bone growth is stimulated at same rate that coating reabsorption.

11. A methos according to claims 1 to 10, wherein the implant to be treated is made of Ti, or any Ti alloy, MgO, Si, GaAs, GeSi, SiC, C, any type of polymer, any type of steel, or any Co-Cr, Al₂O₃ alloy.

12. A material, element, part or implant treated by a method according to claims 1 to 10.
